# EUROPEAN PATENT APPLICATION

(11) **EP 3 498 150 A1**
(43) Date of publication of application: **19.06.2019**
(21) Application number: 17206976.7
(22) Date of filing: 13.12.2017
(51) Int. Cl.: A61B 5/00, G06F 3/01

(54) **HEAD MOUNTABLE APPARATUS**

(71) Applicant: Vestel Elektronik Sanayi ve Ticaret A.S., 45030 Manisa (TR)
(72) Inventor: DÖNER, Çagdas, 45030 Manisa (TR)
(74) Representative: Driver, Virginia Rozanne

(57) **Abstract**

A head-mountable apparatus such as smart glasses has one or more sensors arranged to detect a natural response of a facial feature symptomatic of an illness. An on board processor transmits symptom-related information derived from the sensor to a diagnostic component. An associating method selects a medication to treat a user by capturing image data of medication labels of medicaments available to a user and accessing a database to determine which of the available medicaments is suitable for treating the symptoms. A selected medication notification is generated for display to the user at the smart glasses.

## Description

### Technical Field

The present disclosure relates to head mountable apparatus, particularly but not exclusively of the type known as smart glasses.

### Background

Smart glasses are a form of head mountable apparatus configured to be worn by a human user with a frame configured to be supported by the ears and nose of a human face. They are wearable computer glasses that when worn by a user allow a user to view the world around him through lenses, but also to add information to what the wearer sees. Superimposing information onto a field of view is achieved through an optical head-mounted display (OHMD) or embedded wireless glasses with a transparent heads up display (HUD) or an augmented reality (AR) overlay that has the capability of reflecting projected digital images as well as allowing the user to see through it or to see better with it. Some smart glasses are wearable computer glasses that are able to change their optical properties at runtime. For example, smart sunglasses are programmed to change tint by electronic means.

While early models of smart glasses were able to perform basic tasks, such as serve as a front-end display for a remote system as in the case of smart glasses utilising cellular technology or Wi-Fi, more recently available smart glasses are effectively wearable computers which can run self-contained mobile apps. Some are hands-free and can enable communication with the Internet via natural language voice commands, while others use touch buttons. An 'app' is an abbreviation of an 'application' which is a computer program, often in the form of downloadable code, which can be executed by an on-board computer of the smart glasses. Smart glasses may collect information from internal or external sensors, and can control or retrieve data from other instruments or computers. They may support wireless communication technology such as Bluetooth, Wi-Fi and GPS [Global Positioning System]. Smart glasses may incorporate a camera and other sensors for sensing the environment of a user to provide suggestions and alerts based on what the glasses have sensed in the environment and what has been interpreted by a computer carried by the glasses in that situation.

### Summary

The present disclosure extends the capabilities of smart glasses by equipping them with components and features which enable them to diagnose symptoms of an illness that a wearer might be suffering from, and to enable the recommendation of appropriate medication.

According to a first aspect disclosed herein, there is provided a head-mountable apparatus configured to be worn by a human user, the apparatus comprising:
a frame configured to be supported by the ears and nose of a human face;
sensing circuitry supported by the frame and arranged to detect a natural response of a facial feature of the human user representing a symptom of an illness; and
a processor connected to the at least one sensor and programmed to transmit symptom- related information derived from the sensor to a diagnostic component operable to determine from the symptom-related information if it indicates a symptom of an illness.

The head mountable apparatus may comprise at least one display area supported by the frame and located to be viewed by the user, the display area configured to display information relevant to the illness.

In one embodiment the processor is configured to control the display area to display information from the diagnostic component relating to medication when the symptom-related information indicates that it is indicative of assist symptom of an illness.

The diagnostic component may be provided on the head mountable apparatus, for example as code executed by the processor. Alternatively, the diagnostic component may be a remote diagnostic controller accessible to the processor via a communication network.

In order to detect a natural response of the user's nose when sniffing, for example when the user is suffering from cold or flu, the sensing circuitry may comprise a sensor arranged under a support point of the frame that rests on the bridge of the nose and is arranged to detect natural movement of the nose away from the frame when the user sniffs.

In one embodiment the sensor comprises a piezoelectric sensor which exhibits a change in electro potential indicating that the user has sniffed. It will readily be appreciated that any suitable kind of movement sensor may be used as long as it is capable of detecting natural movement of the nose away from the support point of the frame when the user sniffs.

Another natural response to an illness is that the user's eyes might begin to stream or water. To detect this the sensing circuitry may comprise an image sensor arranged to capture an image of at least one eye of the user and to supply captured image status to the diagnostic component for determining from an eye rinse ratio whether it indicates a symptom of an illness. In this context an eye rinse ratio is the amount that a user's eyes are watering, and can be detected by processing the image data to capture when the eye is watering and when it is dry. In this way it is possible to detect how frequently that eye is watering from the captured image date. Technology for capturing image data and processing it to identify features of an image, such as fluid on an eye, are known.

The processor on board of the head mountable apparatus can be configured to receive the image data from the camera and to process it to supply symptom related information of the image data to the diagnostic component. Alternatively, the image data could be transmitted to a remote diagnostic controller which could carry out processing of the image to determine symptom-related information. That is, the determination of whether or not the image data represents a symptom could be carried out on-board the head mountable apparatus or remotely.

The head mountable apparatus may further comprise a temperature sensor to detect the body temperature of a wearer. In this way it can be determined whether or not the wearer is suffering from a fever, which may be consistent with other detected symptoms of an illness, such as sniffing or watering eyes.

In an enhancement of the smart glasses (head mountable) described herein, features are provided which enable the offering of the wearer to have some medicine that is appropriate to their sniffs, or other symptoms of the illness. The feature enables that a user can go to his pharmacy closet and easily locate what medicines are better for him or her with the aid of the smart glasses. To achieve this, the head mountable apparatus may comprise an outward facing camera which is configured to capture visible medication information from medication labels of medication available to the wearer, and to transmit the medication information to the diagnostic component which is operable to determine therefrom recommended medication appropriate to the symptoms from the medication available to the user.

This is particularly useful because it allows a wearer to select from medicines that the diagnostic controller has determined are available to him and are appropriate for the symptoms that have been detected by the smart glasses.

In accordance with another aspect there is provided diagnostic system comprising:
a head mountable apparatus as defined above; and
a diagnostic controller which is in communication with the head mountable apparatus via a communication network and which comprises the diagnostic component, wherein the diagnostic controller is operable to receive the symptom-related information from the head mountable apparatus and to determine therefrom recommended medication appropriate to the symptoms.

In one embodiment, the diagnostic controller has access to a medication database which holds for a plurality of medications symptoms which may be treated by the respective medication.

The database may further include patient criteria for which the medication is suitable and/or medication periods.

A further aspect provides a method of selecting a medication to treat a user, the method comprising:
capturing image data of medication labels of medicaments available to a user and in the proximity of a user;
determining from apparatus worn by the user that the user is exhibiting one or more symptoms of an illness;
accessing a database to determine which of the available medicaments are suitable for treating the symptoms; and
generating a selected medication notification for display to the user, the notification indicating a medicament suitable to treat a symptom exhibited by the user.

A further aspect provides a computer program product comprising computer code which when executed by a processor implements the method of the preceding paragraph.

### Brief Description of the Drawings

To assist understanding of the present disclosure and to show how embodiments may be put into effect, reference is made by way of example to the accompanying drawings in which:
Figure 1 shows schematically smart glasses capable of detecting symptoms of an illness;
Figure 2 shows schematically a human nose for supporting the smart glasses;
Figure 3 shows schematically a diagnostic system; and
Figure 4 shows exemplary contents of a database.

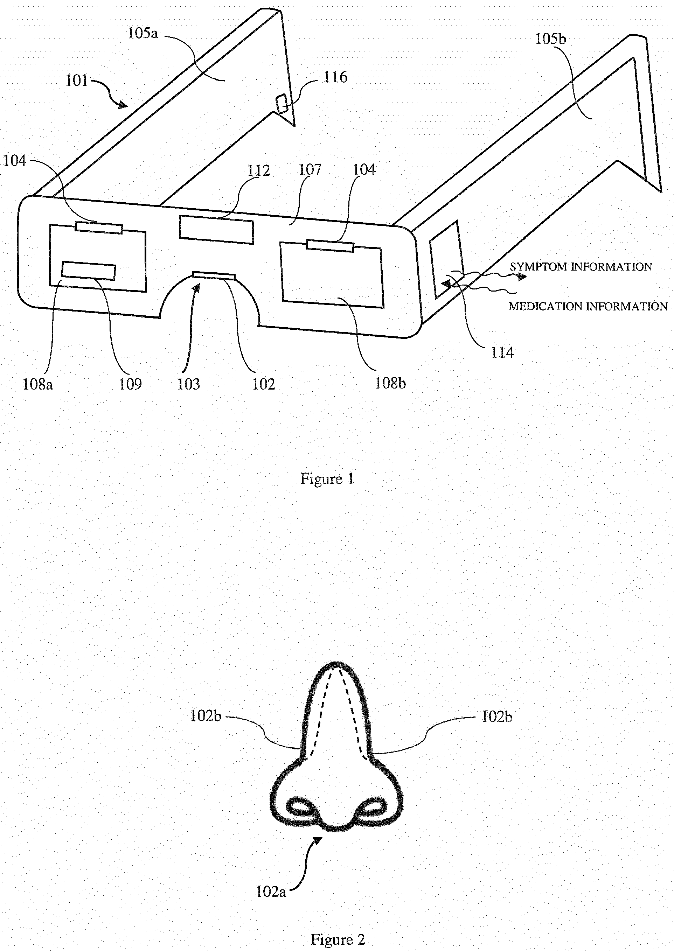

The present disclosure relates to the use of so-called smart glasses in aiding diagnosis of an illness of a wearer of the glasses, and in enabling suitable medication to reduce the symptoms of the illness to be chosen. Figure 1 is a schematic block diagram of smart glasses equipped to aid in the diagnosis of an illness. Reference numeral 101 denotes smart glasses. The smart glasses comprise a frame 105 which has extending earpieces 105a, 105b for supporting the glasses over the ears of a human user. The smart glasses have a central bridge 107 extending between the earpieces 105a, 105b, the central bridge configured to rest on the bridge of a user's nose. The smart glasses have a vision area 108a, 108b for each eye, through which a wearer of the glasses can view his environment. At least one of the vision areas 108a, 108b is equipped with a visible display or projection mechanism which allows a wearer of the glasses to perceive additional information as in the manner of augmented or virtual reality described above. The additional information is presented in area 109 in Figure 1 but it will be appreciated this is highly schematic and any known mechanism for enabling the wearer to view the additional information can be utilised. Such mechanisms are known and will not be described herein.

At least one of the vision areas 108a, 108b is equipped with an image sensor 104 for example in the form of a camera for capturing image data from the eyes of a wearer. These cameras 104 are referred to herein sometimes as internal facing cameras because they face towards the user to capture image data from the user's eyes. The glasses may be further equipped with an outward facing camera 112 which captures an image from the environment of the wearer as will be described in more detail later. The smart glasses comprise an on-board computer 114. The term 'computer' used herein denotes a processor which can execute computer code to implement its functions which provides the smart glasses with local processing capability. The computer 114 is connected to the cameras at 104, 112. The computer 114 has a network interface enabling it to communicate with external components via a communication network for the purposes to be described in more detail later.

The smart glasses further comprise a sensor 102 which acts as a pressure and movement sensor arranged on the glasses' support point 103 on the nose of a user. One or more sensors may be provided. This (or these) sensor(s) 102 operates to detect and monitor the user's nose sniffs. The sniffs can readily be tracked due to the natural movement of the nose as a response to a user sniffing because of the fact that the glasses are supported by the bridge of the nose. When a user sniffs, there will be a clear movement of the nose which is trackable by the sensor or sensors 102. The, or each sensor 102 may be a piezoelectric sensor which can track the pressure exerted at the support point 103 of the glasses which touches the nose. If a user sniffs, his/her nose will be pulled down a little bit so the pressure on the support point will be decreased.

Figure 2 shows a human nose 102a with side parts 102b. As shown by the dotted line in Figure 2, when a user sniffs, the side part contracts slightly to pull the nose down a little away from the sensor 102. A piezoelectric sensor is a suitable choice for the sensor. It is a device that uses the piezoelectric effect to measure changes in pressure or force by converting them to an electrical charge. The voltage output from the sensor is continually monitored by a detection circuit and when a decrease in voltage is measured this can be interpreted as a sniff. The detection circuit may be embodied in the sensor or may be a separate component connected to it or housed in a sensor package. Although not shown in Figure 1, the voltage output derived from the sensor may be connected directly to the computer 114, or to a processing circuit which supplies the computer 114 with detected sniff information. That is, the processing of the raw voltage data from the sensor can be done directly by the on-board computer 114, or there may be an intermediate processing circuit which supplies the computer 114 with information about detected sniffs.

In this way, the smart glasses can be used as a kind of illness tracker by tracking the user's sniffs. If a user repeatedly sniffs, it is possible to recognise that the user may be exhibiting symptoms of an illness, or getting sick.

Another natural response representing a symptom of an illness can be detected by the camera(s) 104 capturing image data from the eye or eyes. A natural response to having a cold or flu is watery eyes. Image data collected from the eye can be processed and analysed to detect how much a user's eye is watering. This is referred to herein as the eye rinse ratio.

The glasses may further be provided with a body temperature sensor 116 to track a user's temperature such that this temperature information may be used in association with the tracked sniffs and/or tracked eye rinse ratio to monitor the user's general health situation. The temperature sensor 116 can be used to detect if the temperature of the wearer is above a certain threshold, indicating a fever.

The smart glasses are thus suitable for monitoring flu or flu like symptoms or other upper respiratory track conditions which affect nose (sniffs), eyes (streaming) and could induce a fever. In some embodiments the output of the camera(s) 104 and/or temperature sensor 116 is supplied to the on-board computer directly or via a processing circuit. The smart glasses described herein enable natural responses of facial features of a human user to be identified as possible symptoms of an illness. The glasses can be used in a context to suggest medication that might be appropriate to ease the wearer's condition. In one embodiment, the on-board computer 114 can detect the indications of symptoms and offer the wearer some healthy activities by way of causing information to be displayed in the display area 109. In another embodiment, symptom data may be transmitted to a remote source and such information, from the computer 114 or from the processing circuits directly.

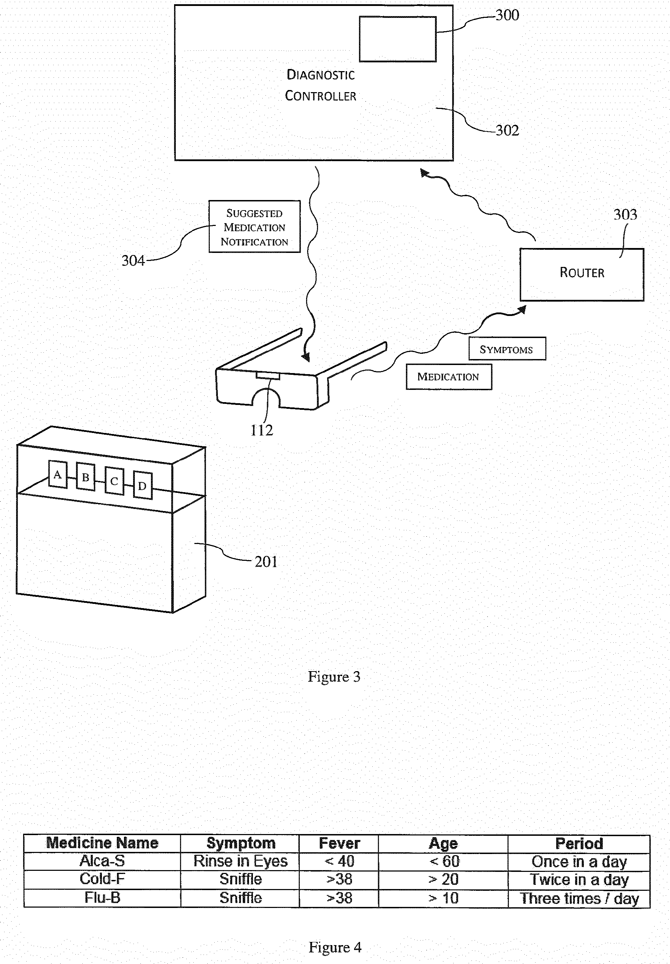

Alternatively, the smart glasses can operate in the context of a diagnostic system which can identify the indicated symptoms and suggest appropriate medication. Figure 3 shows such a system in which a diagnostic controller 302 is in communication with the smart glasses. Figure 3 also illustrates available medication A, B, C, D for the wearer. In this context, available medication means medication which the wearer can readily access. For example, it may be in a pharmacy closet 201 which is in the proximity of the wearer. However, it will be appreciated that the medication could be available in any number of ways. For example, the wearer could be in a store, or could view labelling information for medication over the Internet. The outward facing camera 112 can record image data from labelling information on the available medication and provide that as available medication information to the diagnostic controller 302. The diagnostic controller may be embodied as any suitable computing device executing a suitable program for receiving medication information and for matching it with appropriate symptoms. The symptom information has been supplied from the on-board computer 114 to the diagnostic controller to 302. The diagnostic controller 302 has access to, or includes, a database 300 which stores information associating medications with particular symptoms. Example contents of a database 300 are shown in Figure 4.

The database in Figure 4 is shown in a column structure, but any structure of database could be utilised. The database identifies medicines by name, Alca-S, Cold-F, Flu-B for example. These medications could be considered medication A, B, C which might be available to the user.

Each medication has a symptom that it can be used to treat. Alca-S can be used to treat water in the eyes and a fever less than 40° C. Cold-F can be used to treat sniffles and is suitable for fevers in excess of 38° C. Similarly Flu-B is an alternative medication suitable for the same symptoms. The database includes ages for which the medications are suitable. For example, Alca-S is indicated for people under 60, Cold-S is indicated for adults over the age of 20 and Flu-B is indicated as suitable for children or adults over the age of 10. The database also indicates how often the medication should be taken. For example, Alca-S should be taken once a day, Cold-F should be taken twice in a day and Flu-B should be taken three times a day.

The diagnostics controller receives the medication information captured by the outward facing camera 112; in the case shown in Figure 3 the medication information would identify that medicaments A, B, C and D are all available to the wearer. The diagnostic controller accesses the database 300 based on the available medication information and assesses for what symptoms it is advisable. The diagnostic controller also receives the symptom information and combines this with the medication information to generate a suggested medication notification 304 to be returned to the smart glasses' on-board computer for display to the wearer. The symptoms and medication information may be transmitted from the on-board computer 114 to the diagnostic controller 302 by way of any suitable communications network, for example incorporating a router 303.

In addition to providing an information bar on the display area 109 to suggest a medication to the wearer, it is possible to access the medication's usage periods. Therefore it would be possible to generate alerts for user to cause him to take his/her medication on time.

The connectivity for the glasses with the diagnostic controller 302 could be via the cloud. The smart glasses 101 can establish network connectivity over Wi-Fi or low-power wireless area network (LPWAN) or any other suitable connectivity methods. Reference numeral 303 denotes a connectivity router device for related wireless technology to enable the smart glasses to connect to servers in the cloud which may provide the diagnostic controller.

It will be evident that the diagnostic controller could also be embodied in the on-board computer in some embodiments.

The controller as shown in Figure 3 is represented as a schematic block diagram for the purposes of explaining the functionality of the controller only. Hence, it is understood that the controller may have functional components for performing the functionality ascribed to it herein. Each component may be implemented in hardware, software, firmware, or a combination thereof. Additionally, some or all of the functionality may be performed by a single piece of hardware, software, or firmware.

It will be understood that the processor or processing system or circuitry referred to herein may in practice be provided by a single chip or integrated circuit or plural chips or integrated circuits, optionally provided as a chipset, an application-specific integrated circuit (ASIC), field-programmable gate array (FPGA), digital signal processor (DSP), graphics processing units (GPUs), etc. The chip or chips may comprise circuitry (as well as possibly firmware) for embodying at least one or more of a data processor or processors, a digital signal processor or processors, baseband circuitry and radio frequency circuitry, which are configurable so as to operate in accordance with the exemplary embodiments. In this regard, the exemplary embodiments may be implemented at least in part by computer software stored in (non-transitory) memory and executable by the processor, or by hardware, or by a combination of tangibly stored software and hardware (and tangibly stored firmware). Reference is made herein to data storage (database) for storing data. This may be provided by a single device or by plural devices. Suitable devices include for example a hard disk and non-volatile semiconductor memory.

Although at least some aspects of the embodiments described herein with reference to the drawings comprise computer processes performed in processing systems or processors, the invention also extends to computer programs, particularly computer programs on or in a carrier, adapted for putting the invention into practice. The program may be in the form of non-transitory source code, object code, a code intermediate source and object code such as in partially compiled form, or in any other non-transitory form suitable for use in the implementation of processes according to the invention. The carrier may be any entity or device capable of carrying the program. For example, the carrier may comprise a storage medium, such as a solid-state drive (SSD) or other semiconductor-based RAM; a ROM, for example a CD ROM or a semiconductor ROM; a magnetic recording medium, for example a floppy disk or hard disk; optical memory devices in general; etc.

The examples described herein are to be understood as illustrative examples of embodiments of the invention. Further embodiments and examples are envisaged. Any feature described in relation to any one example or embodiment may be used alone or in combination with other features. In addition, any feature described in relation to any one example or embodiment may also be used in combination with one or more features of any other of the examples or embodiments, or any combination of any other of the examples or embodiments. Furthermore, equivalents and modifications not described herein may also be employed within the scope of the invention, which is defined in the claims.

## Claims

1. A head-mountable apparatus (101) configured to be worn by a human user, the apparatus comprising:
a frame (105a, 105b, 107) configured to be supported by the ears and nose (102a) of a human face;
sensing circuitry (102, 104), supported by the frame and arranged to detect a natural response of a facial feature of the human user representing a symptom of an illness; and
a processor (114) connected to the at least one sensor and programmed to transmit symptom-related information derived from the sensor to a diagnostic component (302) operable to determine from the symptom-related information if it indicates a symptom of an illness.

2. A head-mountable apparatus (101) according to claim 1 comprising at least one display area (109) supported by the frame and located to be viewed by the user, the display area (109) configured to display information relevant to the illness.

3. A head-mountable apparatus (101) according to claim 2 wherein the processor is configured to control the display area (109) to display information from the diagnostic component relating to medication when the symptom-related information indicates that it is indicative of a symptom of an illness.

4. A head-mountable apparatus according to any preceding claim wherein the sensing circuitry comprises a sensor (102) arranged under a support point (103) of the frame that rests on the bridge of the nose of the human user and is arranged to detect natural movement of the nose away from the frame when the user sniffs.

5. A head-mountable apparatus according to claim 4 wherein the sensor comprises a piezoelectric sensor which exhibits a change in electropotential indicative that the user has sniffed.

6. A head-mountable apparatus according to any preceding claim wherein the sensing circuitry comprises an image sensor (104) arranged to capture an image of at least one eye of the user and to supply captured image data to the diagnostic component for determining from an eye rinse ratio whether it indicates a symptom of an illness.

7. A head-mountable apparatus according to claim 7 wherein the processor is configured to receive the image data from the camera (104) and to process the image data to supply symptom-related information of the image data to the diagnostic component.

8. A head mountable apparatus according to any preceding claim comprising a temperature sensor (116).

9. A head mountable apparatus according to any preceding claim comprising an outward facing camera (112) which is configured to capture visible medication information from medication labels of medication available to the user wearing the head-mountable apparatus and to transmit the medication information to the diagnostic component which is operable to determine therefrom recommended medication appropriate to the symptoms from the medication available to the user.

10. A head mountable apparatus according to any preceding claim wherein the processor comprises a network interface operable to transmit symptom-related information to a diagnostic controller (302) via a communication network, the diagnostic controller comprising the diagnostic component.

11. A diagnostic system comprising:
a head mountable apparatus (101) according to claim 9; and
a diagnostic controller which is in communication with the head mountable apparatus via a communication network and which comprises the diagnostic component, wherein the diagnostic controller is operable to receive the symptom-related information from the head mountable apparatus and to determine therefrom recommended medication appropriate to the symptoms from the medication available to the user.

12. A diagnostic system according to claim 11, wherein the diagnostic controller has access to a medication database which holds for a plurality of medications symptoms which may be treated by the respective medication.

13. A diagnostic system according to claim 12, wherein the database includes patient criteria for which the medication is suitable and/or medication periods.

14. A method of selecting a medication to treat a user, the method comprising:
capturing image data of medication labels of medicaments available to a user and in the proximity of a user;
determining from apparatus worn by the user that the user is exhibiting one or more symptoms of an illness;
accessing a database to determine which of the available medicaments are suitable for treating the symptoms; and
generating a selected medication notification for display to the user, the notification indicating a medicament suitable to treat a symptom exhibited by the user.

15. A computer program product comprising computer code which when executed by a processor implements the method of claim 14.
